# EUROPEAN PATENT SPECIFICATION

(11) **EP 2 211 941 B1**
(45) Date of publication and mention of the grant of the patent: **21.11.2018**
(21) Application number: 08833938.7
(22) Date of filing: 23.09.2008
(51) Int. Cl.: A61M 5/168

(54) **INJECTION MONITOR**
INJEKTIONSMONITOR
MONITEUR D'INJECTION

(30) Priority: 24.09.2007 US 974495 P
(43) Date of publication of application: 04.08.2010
(73) Proprietor: Liebel-Flarsheim Company LLC, Cincinnati, OH 45237 (US)
(72) Inventor: POOLEY, David, M., Stapleford Cambridgeshire CB22 5EB (GB); LAITENBERGER, Peter, G., Cambridge, Cambridgeshire CB4 1AG (GB)
(74) Representative: Edson, Russell Gregory
(86) International application number: PCT/US2008/077349
(87) International publication number: WO 2009/042577

(56) References cited:
- WO-A-99/27981
- GB-A- 2 396 221
- GB-A- 2 426 586
- US-A- 5 935 105
- US-B1- 7 047 058

## Description

### FIELD OF THE INVENTION

The invention relates to a passive injection monitoring device.

### BACKGROUND

This section is intended to introduce the reader to various aspects of art that may be related to various aspects of the present invention, which are described and/or claimed below. This discussion is believed to be helpful in providing the reader with background information to facilitate a better understanding of the various aspects of the present invention. Accordingly, it should be understood that these statements are to be read in this light, and not as admissions of prior art.

Generally, a power injector is used to inject medical fluid, such as a pharmaceutical (e.g., radiopharmaceutical) or a contrast agent, into a patient. Typically, the power injector injects the medical fluid into the patient via a catheter that is disposed under the skin of an arm of the patient. During the injection, the medical fluid passes through the catheter and into a vein or other desired location within the patient.

Unfortunately, not all injections proceed correctly. For instance, during an injection, medical fluids may enter the surrounding tissue, either by leakage (e.g., because of brittle veins in very elderly patients), or direct exposure (e.g., because the needle has punctured the vein and the infusion goes directly into the arm tissue). This is often referred to as "extravasation." In mild cases, extravasation can cause pain, reddening, or irritation on the arm with the syringe. If uncorrected, extravasation can lead to other medical complications. With power injectors that inject medical fluids at a high rate, it may be more likely that more medical fluid is injected into the patient before the symptoms of extravasation are identified and corrective action can be taken. GB-A-2426586 discloses a microphone for detecting the acoustic signal generated by blood flowing in a blood vessel.

### SUMMARY

The invention relates to a passive injection monitoring device. All of the following embodiments relate only to passive acoustic injection monitoring devices, even if not explicitly mentioned.

It should be understood that these aspects are presented merely to provide the reader with a brief summary of certain forms the invention might take. Indeed, the invention may encompass a variety of aspects that may not be set forth below.

The present invention relates to an injection monitor that includes passive acoustic sensors (e.g., microphones) that sense noises (e.g., acoustic emissions) produced by the flow of a medical fluid that has been injected into a subject (e.g., a patient). The injection monitor may process the sensed noises to determine if a problem, such as an extravasation, has occurred. In certain embodiments, the injection of the medical fluid into a subject is controlled based on processing of the sensed noises and whether a problem has occurred as indicated by the sensed noises.

In accordance with a first embodiment of the present invention, there is provided an extravasation detector, wherein the detector is further connected to a monitor comprising a memory. In accordance with a second embodiment of the present invention, there is provided a medical fluid injector, comprising a power injector, and the extravasation detector of the first embodiment configured to monitor acoustics associated with an injection of a medical fluid by the power injector.

In accordance with other arrangements described herein, there is provided a method of monitoring for extravasation, comprising sensing acoustic emissions produced by fluid flow of a medical fluid injected into a subject, and detecting a possible extravasation event based on the sensed acoustic emissions.

Arrangements described herein may comprise an injector, comprising an injector, and an acoustic sensor.

The injection monitor may be configured to monitor acoustic emissions associated with an injection and to determine if an extravasation event has occurred, is occurring, or may occur.

In accordance with other arrangements described herein, there is provided a tangible medium, comprising a machine readable medium, and code disposed on the machine readable medium, wherein the code is configured to monitor acoustic emissions associated with an injection and to determine if an extravasation event has occurred, is occurring, or may occur.

Various refinements exist of the features noted above in relation to the various aspects of the present invention. Again, the brief summary presented above is intended only to familiarize the reader with certain aspects and contexts of the present invention without limitation to the claimed subject matter.

Extravasation detectors are known in the prior art, for example from patent document US 7,047,058. The detectors comprise active parts, like an energy source.

### BRIEF DESCRIPTION OF THE DRAWINGS

These and other features, aspects, and advantages of the present invention will become better understood when the following detailed description is read with reference to the accompanying drawings in which like characters represent like parts throughout the drawings, wherein:
FIG. 1 is a diagram of an embodiment of an injection system;
FIG. 2 is a diagram of an alternate embodiment of an injection system;
FIG.3 is a perspective view of an embodiment of a power injector;
FIG.4 is a flow chart of an extravasation detection process;
FIG. 5 is a flow chart of an injection control process; and
FIG. 6 is a flow chart of another extravasation detection process.

### DETAILED DESCRIPTION OF SPECIFIC EMBODIMENTS

One or more specific embodiments of the present invention will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a", "an", "the", and "said" are intended to mean that there are one or more of the elements. The terms "comprising", "including", and "having" are intended to be inclusive and mean that there may be additional elements other than the listed elements. Moreover, the use of "top", "bottom", "above", "below" and variations of these terms is made for convenience, but does not require any particular orientation of the components.

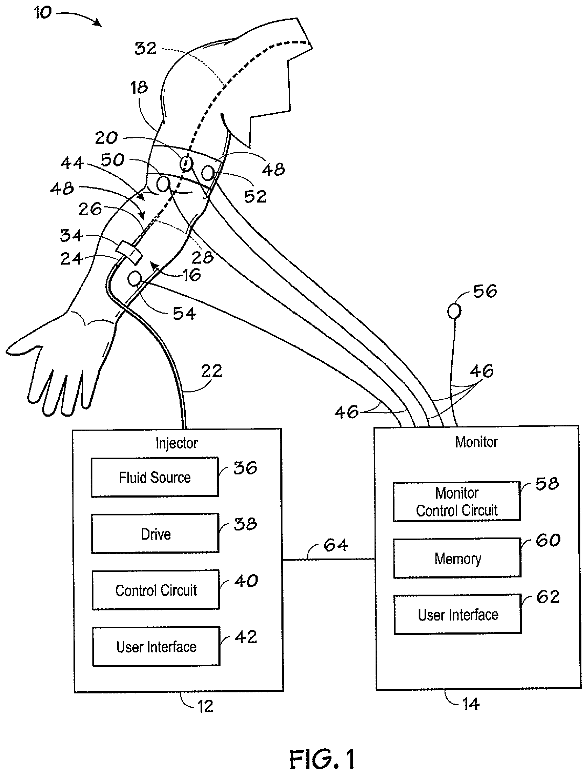

FIG. 1 illustrates an exemplary injection system 10 having an injector 12 and a monitor 14. Further, the system 10 includes a catheter 16 that may be inserted into a patient 18 and a sensor 20 that can detect acoustic emissions. Advantageously, certain embodiments that are discussed in detail below may include disposing the catheter 16 for injection of a medical fluid into the patient 20, and disposing the sensor 20 such that the sensor 20 can sense acoustic emissions generated when the medical fluid is injected into the patient 18. The monitor 14 may receive signals indicative of the sensed emissions and process the signals to determine whether the medical fluid is being delivered properly (e.g., whether extravasation has occurred, is occurring, or may occur). In certain embodiments, processing may include comparing the sensed emissions to baseline emissions that are acquired under known conditions, such as a baseline acquired prior to the injection. In other words, the baseline may correspond to acoustic emissions for normal blood flow without an injection and/or a proper injection with extravasation. The baseline may be based on normal blood flow and/or a proper injection of the specific patient 18, an average over a group of patients or test subjects, or a theoretical/mathematical model, or a combination thereof. The baseline also may be based on normal blood flow and/or a proper injection in the same region of tissue, e.g., an arm. Further, embodiments may include controlling the injection based on whether an extravasation has occurred, is occurring, or may soon occur based on acoustic emissions.

In certain embodiments, one or more sensors 20 may be placed at various locations, including on an arm band, proximate to a vein with the medical fluid is injected therein, upstream of the injection site, down stream of the injection site, on tissue proximate to the vein and/or the syringe, or various locations where the acoustic emissions can be sensed by the sensor 20. These sensors 20 may be separate from one another and other components. In certain embodiments, the sensor 20 is coupled to the catheter 16, such that placement of the catheter 16 and the sensor 20 may be simplified. For example, the catheter 16 may have an integral sensor 20 or an add-on sensor 20, such as a retrofit sensor 20 adapted to fit onto a variety of standard catheters 16. Further, in certain embodiments, the injector 12 and the monitor 14 may be combined to provide a compact unit capable of injecting medical fluids and detecting extravasation events, for instance.

FIG. 1 illustrates a system 10 for injection of a medical fluid into the patient 20 and for detecting extravasation events that may result from the injection. During an injection, the injector 12 pressurizes a medical fluid that is delivered to the patient 18 via an injection tube 22 and the catheter 16. As illustrated, the catheter 16 provides for a termination of the injection tube 22 into the patient 18. Generally, the catheter may include a body 24, a sheath 26 and a needle 28. The body 24 of the catheter 16 may include a housing or general structure that provides rigidity for handling by a clinician and protection of the sheath 26 and the needle 28. For example, the body 24 may include a plastic structure that houses the sheath 26 and the needle 28 and includes a location for the clinician to handle the catheter 16. The catheter 16 may include various configurations. For example, the catheter 16 may consist only of the sheath 26 and the needle 28.

The sheath 26 of the catheter 16 may generally include a structure that is inserted into a vein 32 of the patient 18. Once inserted, the sheath 26 may provide a channel for the flow of the medical fluids into the patient 18. For example, in one embodiment, the catheter 16 includes a Teflon sheath 26 that surrounds the needle 28. Generally, the catheter 16 is inserted into the patient 18 by puncturing the patient's 18 skin and vein 32 with the needle 28, and subsequently threading the sheath 26 into the vein 32. The needle 28 may then be removed, leaving the sheath 26 to provide for a path to deliver the medical fluids into the vein 32 of the patient 18. Once the catheter 16 is inserted into the patient 18, the injection tube 22 may be connected to the catheter 16. Further, an adhesive pad 34 may be used to secure the catheter 16 to the patient 18. In other embodiments, the needle 28 may remain a part of the catheter 16 after insertion into the patient 18. For instance, the needle 28 may be used to puncture the patient's 18 skin and a vein 32, and medical fluids may be injected into the patient 18 via a hollow channel running the length of the needle 28.

As discussed previously, the injector 12 may provide a source of the medical fluid injected into a patient via the injection tube 22 and the catheter 16. The injector 12 may include various injection mechanisms, including a powered injector. Generally powered injectors 12 may provide a steady flow of medical fluids at various flow rates. For example, an injector 12 may provide for flow rates ranging from 0.1 to 10 milliliters (mL) per second, and delivery pressures such as 50-325 PSI (pounds per square inch). The automated nature of powered injectors 12 may provide for accurate delivery of medical fluids to the patient 18. Further, powered injectors 12 may include various settings and features to increase flexibility of the system. For example, a powered injector 12 may include various checks (e.g., patency checks) and various modes and protocols for different injection types.

In the illustrated embodiment, the injector 12 includes a powered injector having a fluid source 36, a drive 38, a control circuit 40, and a user interface 42. The components of the injector 12 generally act in cooperation to deliver the medical fluid from the injector 12. The fluid source 36 may include a container (e.g., a syringe) that houses medical fluids (e.g., a contrast agent, a pharmaceutical, a radiopharmaceutical, saline, or a combination thereof), In one embodiment, the injector 12 may have multiple fluid sources 36. For example, the fluid source 36 may include a first syringe filled with a contrast agent and a second syringe filled with a saline solution. In an embodiment that includes a syringe as a fluid source 36, the medical fluid is generally injected directly from the syringe to the injection tube 22. For example, a syringe generally includes a plunger, a barrel, and the medical fluid disposed within the barrel. To eject the medical fluid from the syringe, the plunger may be moved along the length of the barrel, causing the medical fluid to be pushed out of a tip of the syringe and into the injection tube. Generally, the plunger is moved by the drive 38.

The drive 38 generally includes a mechanism to force the medical fluid from the fluid source 36 into the injection tubing 22. For example, in an embodiment including a syringe as the fluid source 36, the drive 38 may include an electric motor that drives a ram that, in turn, moves the plunger of the syringe through the barrel of the syringe, and pushes medical fluid out of the syringe and into the patient 18. In other embodiments, the drive 38 may include other devices or mechanisms configured to force medical fluid from the fluid source 36 and into the injection tube 22. For example, in another embodiment, the drive 38 may include a pump configured to pump the medical fluid from the fluid source 36 and into the injection tube 22.

The control circuit 40 of the injector 12 may generally include circuitry that is capable of receiving various inputs and controlling operation of the injector 12 based on the inputs and various other parameters. For example, operation of the drive 38 and, thus, the injection of the medical fluid from the fluid source 36, may be controlled by the control circuit 40 of the injector 12. In one embodiment, the control circuit 40 may provide signals that regulate operation of the drive 38, and, thus, regulate the flow of medical fluids from the fluid source 36.

Inputs to the control circuit 40 may include various feedback signals and parameters, such as those entered by a clinician. For example, the control circuit 40 of the injector 12 may be in communication with the user interface 42. Generally, the user interface 42 may include various inputs and outputs, such as knobs, dials, a touch screen LCD and the like that are accessible by a user. For example, in one embodiment, the user interface 42 includes a touch screen LCD that includes inputs for injection parameters, including the desired injection flow rate, injection volume, injection protocols, and the like. Further, the LCD touch screen may include visual feedback to the user, including parameters such as the fill volume of the syringe, patency check, current selections, and progress of the injection. The inputs from the user interface 42 may be transmitted to the control circuit 40 to coordinate user request with operation of the injector 12. For example, a clinician may select a given flow rate that is received by the control circuit 40, and, in response, the control circuit 40 may signal the drive 38 to operate such that the medical fluid is delivered at the desired rate.

During an injection procedure, medical fluids may be delivered to the patient 18 from the injector 12 via the injection tube 22 and the catheter 16, as discussed previously. It may be desirable to monitor the progress of injections to ensure that they are proceeding correctly. For instance, during an injection, medical fluids may enter the surrounding tissue, either by leakage (e.g., because of brittle veins in very elderly patients), or direct exposure (e.g., because the needle has punctured the vein and the infusion goes directly into the arm tissue) resulting in an extravasation. In mild cases, extravasation can cause pain, reddening, or irritation on the arm with the syringe. If uncorrected, extravasation can lead to other medical complications. With powered injectors that are capable of injecting medical fluids at a high rate, such as greater than 3 mL per second, it may be more likely that an increased amount of medical fluid is injected into the patient before the symptoms of extravasation are identified and corrective action can be taken. Rapid detection of any unusual behavior and symptoms may be used to identify extravasation and enable a system or care provider to take appropriate action to minimize the likelihood of patient discomfort and potential complications.

Generally, as an injection proceeds, characteristic sounds may be generated by the fluid flow of the medical fluids, blood and other bodily fluids. Thus, an injection that is proceeding correctly may generate characteristic sounds (i.e., an acoustic signature) of fluid flow through a given path, such as the vein 32. However, if a problem occurs, such as extravasation, the generated acoustic signature may vary and, thus, be indicative of the incorrect flow of the medical fluid. Provided below is an injection system 10 that includes an injection monitoring device that uses passive acoustic devices (e.g., a microphone) and methods to monitor the progress of an automatic infusion or injection process.

As illustrated in FIG. 1, an embodiment of the system 10 includes the sensor 20 and the monitor 14. Generally, the sensor 10 may sense the acoustic emissions being generated during an injection process and transmit signals indicative of the acoustic emissions to the monitor 14. Based on the emissions, the monitor 14 may determine if the injection is proceeding correctly, or if a fault, such as extravasation, has occurred, is occurring, or may occur. In one embodiment, the monitor 14 may transmit this determination and other information to components of the system 10, such as the injector 12, to control the injection accordingly.

The sensor 20 may include any passive device that is capable of sensing acoustic emissions, such as those generated during injection of a medical fluid into a patient 18. In one embodiment, the sensor 20 may include a microphone. In such an embodiment, the microphone may include a sensitivity that is sufficient to sense and transmit the acoustic emissions generated by the fluid flow of blood and/or medical fluid in the vein 32 or a surrounding tissue 44. As depicted, the sensor 20 may include a wired connection to the monitor 14 via a sensor cable 46. In other embodiment, the sensor 20 may include a wireless configuration, such that the sensor 20 may transmit signals to the monitor 14 without the use of the sensor cable 46.

The sensor 20 may be affixed to the patient 18 by various mounts to enable the sensor 20 to sense the acoustic emissions. For example, as illustrated in FIG. 1, the sensor 20 may be coupled to the patient 18 via an arm band 48. The arm band 48 may include a material that is stretched around the arm or other location on the patient 18 to provide contact between the sensor 20 and the skin of the patient 18. In another embodiment, the sensor 20 may be secured to the patient 18 via an adhesive patch. For example, as depicted, a sensor 50 may be coupled directly to the skin of the patient 18. In yet another embodiment, affixing the sensor 20 to the patient 18 may include the use of a disposable acoustic matching layer disposed between the sensor 20 and the patient 18, such that the sensor 20 may be reused without the need to be sterilized after each use.

FIG. 1 also illustrates embodiments of the system 10 that include various positions of the sensor 20 and the use of a plurality of sensors 20. In one embodiment, placement of the sensor 20 may include locating the sensor 20 proximate to the catheter 16 and an injection site 48, to enable the sensor 20 to sense the acoustic signals generated by fluid flow within the patient 18. For example, as depicted, the sensor 20 may be disposed on the patient "in-line" with the vein 32. In other embodiments, the sensor 20 may be placed at various locations on or near the patient 18. For example, as illustrated, a sensor 52 may be disposed on the arm band 48 and not in-line with the vein 32. Such an arrangement may provide for increased sensitivity in detecting acoustic emissions generated by fluid flow in the tissue 44 surrounding the vein 32. Similarly, as illustrated, the sensor 50 may be disposed directly to the patient 18 and proximate to the surrounding tissue 44. Further, embodiments may include positioning the sensor 20 downstream or upstream of the fluid flow of the injection. For example, the system 10 may include a sensor disposed downstream of the injection site 48, such as sensors 20, 50, 52, and/or a sensor 54 disposed upstream of the injection site 48.

The system 10 may also include a plurality of sensors disposed simultaneously on or near the patient 18. The use of a plurality of sensors may enable the system 10 to account for various factors in the detection of an extravasation event. For example, the use of multiple sensors may increase the sensitivity of the system 10 such that minute variations are sensed and processed. Further, multiple sensors may enable the system 10 to monitor a larger area and volume of the patient 18. For example, an embodiment including the sensor 20 in line with the vein 32 and an additional sensor 50 and/or 52 proximate to the surrounding tissue 44 may enable the system 10 to detect fluid flow characteristics within the vein 32 as well as fluid flow in the tissue 44 (e.g., medical fluid leaking into the tissue 44). In another embodiment, the inclusion of multiple sensors may enable the system 10 to more accurately locate the extravasation. For example, acoustic emissions gathered from three or more sensors may enable the system 10 to determine the position of the extravasation by triangulation.

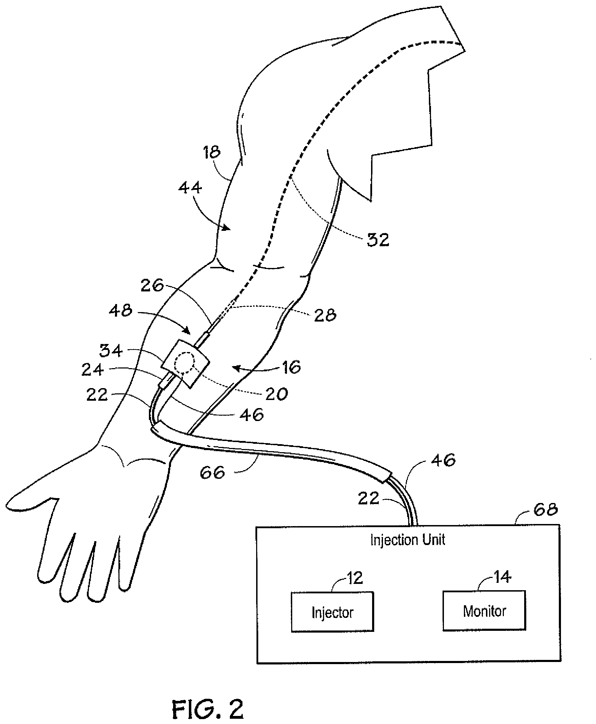

Further, the effects of background noise may be reduced by employing at least a second sensor. In one embodiment, a second sensor may be disposed to sense acoustic noises generated in an area surrounding the patient 18. For example, as illustrated in FIG. 2, a second sensor 56 may be located a distance from the patient 18 such that the sensor 56 senses noise generated in the area surrounding the patent 18. In processing, the acoustic emissions sensed by the second sensor 56 may be subtracted from the acoustic emissions sensed by the primary sensor 20, 50, 52, and/or 54. Subtracting the two signals may enable the cancellation of noise and, thus, increase the accuracy of the detection of a fault condition, such as extravasation.

As discussed briefly above, signals from the sensors 20, 50, 52, 54, and/or 56 may be transmitted to the monitor 14 via the sensor cable 46 or wirelessly. Generally, the monitor 14 may include various components to condition and process the signals transmitted by the sensors as well as various outputs that may be transmitted to a user clinician or other components within the system 10. For example, as illustrated, the monitor 14 includes a monitor control circuit 58, a memory 60 and a user interface 62.

The monitor control circuit 58 may be configured to condition the signals that are received from the sensors. For example, the monitor control circuit 58 may include band-pass filters or comparators to cancel any extraneous noise that may be present in the transmitted signal. Further, the control circuitry 58 may include a processor that employs signal processing to determine if an extravasation event has occurred, is occurring, or may occur. For example, the processor may perform spectral analysis with a series of electronic filters, a Fourier transform, a wavelet transform, and the like procedure.

Embodiments of the monitor 14 may also include the memory 60. For example, various routines and procedures may be stored on the memory 60 and retrieved by the processor during operation. In one embodiment, the monitor 14 may store acoustic signatures in memory 60 and compare the acoustic emissions acquired during an injection to the stored acoustic signatures to determine if an injection is proceeding correctly or not. For example, the monitor may store one or multiple acoustic signatures (e.g., "baselines") in memory 60 for successful and unsuccessful injections, and compare the sensed acoustic emissions to each of the stored acoustic signatures to determine if an extravasation has occurred and the extent of the extravasation. In one embodiment, the baseline acoustic signature stored in memory 60 may include acoustic emissions captured proximate to the time of the injection. For example, prior to injection of the patient 18 with the medical fluid, the monitor 14 may capture and store an acoustic emission indicative of normal blood/fluid flow.

In addition to making a determination of whether an injection is proceeding correctly, the monitor 14 may include an output that is indicative of the characteristics of the injection. For example, the monitor 14 may include an output to the clinician or other components of the system 10. In one embodiment, the monitor 14 may output an indication of injection status to a user interface 62. The user interface 62 may include an LCD screen, and alarm, or other visual or audible indicator. In one embodiment, the monitor 14 may also transmit information regarding the status of the injection to other components of the system 10, such as the injector 12. For example, the monitor 14 may transmit the status to the injector via a cable 64. In one embodiment, the injector 12 may control the injection of medical fluids into the patient 18 based on the status. For example, if an extravasation is detected, the monitor 14 may output a signal that is indicative of the condition to the injector 12, and the injector 12 may terminate or modify the injection based on the indication of the extravasation. The system 10 may also base its response on other parameters, such as the progress of the injection.

FIG. 2 illustrates an embodiment of the system 10 that includes the sensor 20 disposed proximate to the catheter 16. In one embodiment, the sensor 20 may be disposed on the body 24, sheath 26 or needle 38 of the catheter 16. For example, the sensor 20 may include a component that is adhered to the catheter 16 via and adhesive patch 34 or other coupling, such as an epoxy adhesive, or a mechanical clip. In another embodiment, the sensor 20 may be manufactured as an integral component of the catheter 16. Further, the depicted sensor cable 46 and the injection tube 22 are contained in a single cable sheath 66. Advantageously, coupling the catheter 16 proximate to the catheter 16 may enable a clinician to affix both the catheter 16 and the sensor 20 to the patient 18 simultaneously. Such an arrangement may also ensure that the sensor 20 is accurately disposed on or near the patient 18. For example, the sensor 20 may be affixed such that it is located a given distance from the tip of the sheath 26.

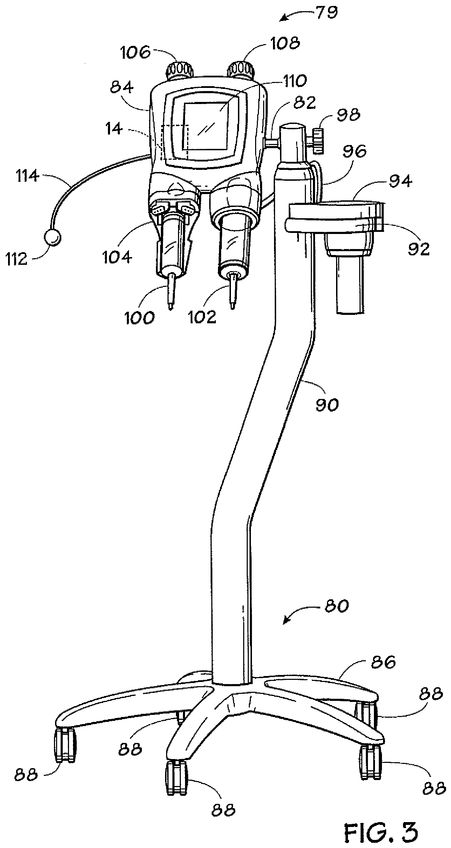

Further, FIG. 2 illustrates a single injection unit 12 that includes both the injector 12 and monitor 14. As described previously, the injector 12 and the monitor 14 may provide for injection of the medical fluid and monitoring the injection for extravasation, respectively. Accordingly, the injection unit 68 may provide for the combined functionality in a compact form factor. For example, in one embodiment, as discussed in greater detail below with regard to FIG. 3, the injection unit 68 may include a single power head of an injector system. Accordingly, a clinician may only need to operate a single device to provide for injection of a medical fluid and monitoring of the injection. Other embodiments may include various features discussed previously, including employing multiple sensors, employing multiple sensor locations, interactions via a user interface, and the like.

During injection, fluid flowing through the needle 28 and/or the sheath 26 may produce additional acoustic emissions that are sensed by the sensors. In one embodiment, the acoustic emissions associated with the fluid flow through the needle 28 or sheath 26 may be manipulated with the addition of features to control the acoustic emission. For example, the needle 28 or sheath 26 may include periodic ridges to actively generate a fundamental frequency proportional to flow rate. Features may also be included to provide longitudinal resonances. In another embodiment, mechanical resonating structures may be included to produce other acoustic patterns. For example, reeds or other vibrating mechanisms may be included to generate vibrations that can be sensed by the sensors. In certain embodiments, the vibrating mechanism may be switched on and off, for example, using an external electromagnet. Changes in the sound may correspond to changes in the injection procedure (e.g., spectral characteristics).

FIG. 3 is an elevation view of an exemplary powered injector 79. As illustrated by FIG. 3, the powered injector 79 includes a stand assembly 80, a support arm 82, and a power head 84. The illustrated stand assembly 80 includes a pedestal 86, wheels 88, a vertical support 90, a handle 92, and a rack 94. As illustrated, a power cable 96 is routed internal to the vertical support 90 and terminates into the power head 84. The power head 84 is coupled to the stand assembly 80 via the support arm 82. The support arm 82 includes ball and socket connection between the power head 84 and the stand assembly 80 such that adjustment of a knob 98 may provide for movement in two degrees of freedom. For example, the power head 84 is rotatable about the axis of the support arm 82 such that the power head 84 may be rotated from the illustrated position, e.g., the power head 84 facing downward, to a horizontal position, and/or a position with the power head 84 tilted upward.

The power head 84 of the injector 79 shown in FIG. 3 may include components of the injector 12, as discussed with reference to FIG. 1. For example, the power head 84 includes a medium syringe 100 and a saline syringe 102. In one embodiment, the medium syringe 100 may be filled with medical fluids such as a contrast agent, a pharmaceutical, a radiopharmaceutical, saline, or a combination thereof. Each of the syringes 100, 102 may include a fluid capacity, such as 200 mL. During operation, the medium syringe 100 may be employed to inject the patient 18 with the medical fluid, and the saline syringe 102 may be employed to inject a saline solution to flush the medical fluid through an injection tube connected between the syringes 100, 102 and a catheter inserted into the patient 18. Operation of the syringes 100, 102 may be similar to those discussed previously. For example, one or more drive units within the power head 84 may be employed to drive a ram that pushes the plunger of each syringe 100, 102 to eject the medical fluid out of the syringes 100, 102.

The power head 84 also includes a heater blanket 104 that surrounds the medium syringe 100. The heater blanket 104 may be employed to heat the medical fluids contained in the medium syringe 100 to approximately body temperature before injecting the medical fluid into the patient 18. In one embodiment, the heater blanket 104 may automatically recognize the volume of medical fluid in the syringe 100 and adjust operation of the heater blanket accordingly 104.

Manual flow knobs 106, 108 are also provided. The manual control knobs 106, 108 may provide for manual adjustment of the plunger of each syringe 100, 102. Accordingly, the manual control knobs 106, 108 may be rotated to advance the plunger to expel a given amount of medical fluid from one of the syringes 100, 102. For example, a clinician may rotate the manual control knob 106, causing the plunger of the medium syringe 100 to push the medical fluid out of the syringe 100. This may be particularly useful for flushing a medical fluid through a medical tubing 22 prior to an injection procedure.

The illustrated power head 84 also includes a display 110. In one embodiment, the display includes a liquid crystal display (LCD). Further, an embodiment of the display 110 may include a touch-screen that enables a clinician to directly input parameters and settings. For example, a clinician may select the current injection protocol, start an injection, stop an injection, or perform other related functions. In other embodiments, the display 110 may include a cathode ray tube display, and organic light emitting diode display, a surface emission display, or other appropriate display, and it may be coupled to a control circuit 40 within the power head 40.

The power head 84 of the power injector 79 illustrated in FIG. 3 also houses the monitor 14. The monitor 14 is in communication with a sensor 112 via the sensor cable 114 as illustrated. Accordingly, the powered injector 79 is capable of injecting a patient 18 with a medical fluid and monitoring the injection for extravasation.

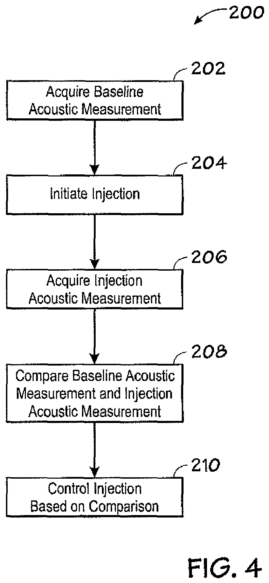

The injection system 10 may operate according to an exemplary injection process 200 depicted by FIG. 4. As depicted by block 202, the injection system 10 first acquires a baseline acoustic measurement. In some embodiments, the baseline acoustic measurement may include previously acquired data, or may include data acquired proximate to the injection procedure. For example, during this step the baseline acoustic measurement may include an acoustic profile that is retrieved from the memory 60. In such an embodiment, the acoustic profile may include data that is representative of an ideal injection process, an exemplary profile of an extravasation, or data indicative of previously sensed and recorded data. In an embodiment, that includes data acquired proximate to the time of the injection, the baseline acoustic measurement may include data indicative of normal blood flow in a patient prior to the start of the injection process. For example, the system 10 may monitor and record data sensed by the sensors just a few minutes before the injection process, and store the profiled in memory 60 for comparison to the acoustic profile sensed during the injection process.

Next, the exemplary injection process 200 includes initiating the injection, as depicted at block 204. In some embodiments, initiating the injection may include a clinician manually initiating an injection process, or the system 10 automatically starting the injection once setup of the system 10 has been verified. For example, one embodiment may include a clinician manually initiating the injection by pressing a start button located on the user interface 42, 62. In another embodiment, the system 10 may automatically proceed to initiate the injection once the baseline acoustic measurement is acquired (block 202). Other embodiments may include the system 10 and/or the clinician performing various checks to verify setup when the injection in initiated. The physical act of initiating the injection may include the drive 38 of the injector 12 moving the plunger of the syringe such that medical fluid is expelled through the medical tube 22. In certain embodiment, the initiation of the injection may also include injecting a saline solution in cooperation with the medical fluid.

During the injection process 200, the system 10 may acquire injection acoustic measurements, as depicted at block 206. Acquiring the injection acoustic measurements may generally include the sensors 20 sensing the acoustic emissions of fluid flow within the patient during the injection, and transmitting signals indicative of the sensed acoustic emissions to the monitor 14 for processing. As discussed previously, the system 10 may include a single sensor 20 or a plurality of sensors disposed at various locations on or proximate to the patient. Thus, acquiring injection acoustic measurements may include acquiring signals from any number of the sensors employed in the system 10. Further, transmission of the signals may be provided via a cabled connection or a wireless connection, as discussed previously.

As depicted at block 208, the injection system 10 compares the baseline acoustic measurement and injection acoustic measurement. In certain embodiments, comparing the baseline may include processing the sensed signal within the monitor 14. For example, the processor within the monitor control circuitry 58 may perform a Fourier transform, a wavelet transform, and the like procedure. In some embodiments, comparing the measurements may include subtracting one signal from the other to identify characteristics of the injection acoustic measurement. For instance, as discussed previously, various acoustic profiles in memory and those profiles detected may be considered in processing to reduce the effects of noise, and to readily identify characteristics of the acquired injection acoustic measurement. The comparison (block 208) of the baseline (block 202) and the injection acoustic measurement (block 206) may provide an indication as to the amount of extravasation, the location of the extravasation, and the like. The results of comparing the measurements (block 208) may be provided to various locations and components of the system 10, including the injector 12 and the user interface 42, 62.

Based on the comparison of the baseline acoustic measurement and the injection acoustic measurement at block 208, the system 10 may then control the injection based on the comparison, as depicted at block 210. In one embodiment, the system 10 may determine that the injection should be terminated or may determine that the injection should continue. For example, upon detection of an extravasation in step 208, the system 10 may consider various factors, including the percentage of the injection that is complete, the extent of the sensed extravasation, and output a signal to the control circuit 40 to continue the injection, modify the injection procedure, or to terminate the injection. In response, the drive 38 may remain engaged to continue the injection, may drive the plunger of the syringe at a different rate, or may be disengaged to terminate the injection. In each of the embodiments, the system 10 may also provide feedback to the user via a user interface 42, 62 and may take steps to automatically control the injection or enable manual control of the injection, based on the configuration of the system 10.

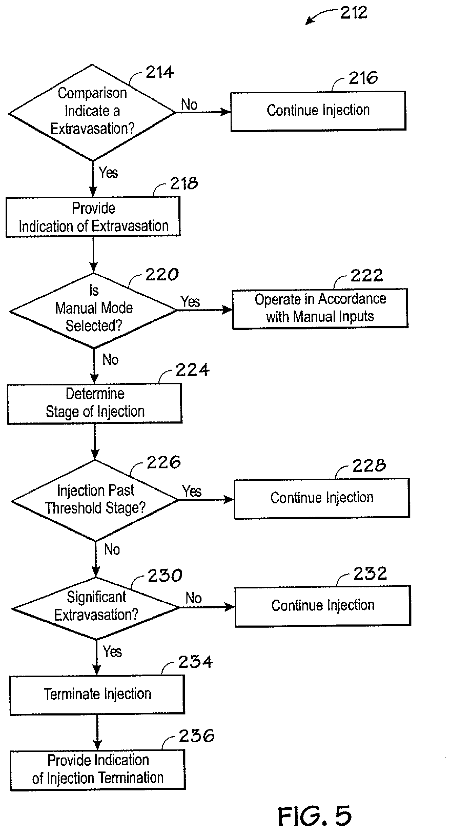

FIG. 5 depicts a detailed embodiment of a control process 212 for an injection based on a comparison of a baseline with injection acoustic measurements as discussed above with reference to FIG. 4. For example, as depicted at block 214 of FIG. 5, the system 10 may first determine if the comparison at block 208 of FIG. 4 indicates an extravasation. If the comparison does not indicate an extravasation, the system 10 may continue the injection, as depicted at block 216. In one embodiment, continuing the injection may include returning to block 206 of FIG. 4 and continuing to acquire an injection acoustic measurement and proceeding through the steps at blocks 208, 210 and 214 to monitor the injection process. If the comparison does indicate an extravasation, the process 212 may provide an indication of extravasation, as depicted at block 218. For example, upon the determination that an extravasation has occurred, the system 10 may provide an audible alert and/or a visual alert via the user interface 42, 62. The indication may also include relevant information, including the location of the extravasation, the extent of the extravasation, the percentage of the injection complete, and the like.

Subsequent to determining there is an extravasation (block 214) and providing an indication of extravasation (block 218), it may be determined if a manual mode has been selected, as depicted at block 220. In other words, the system 10 may determine if a clinician has selected automatic control of the injection upon detection of an extravasation, or if the clinician has elected to control the system 10 manually when an extravasation has occurred. If manual mode has been selected, upon determination that an extravasation has occurred, the system 10 may provide an indication of the extravasation (block 218) and, then, enable the system 10 to continue operation in accordance with manual inputs, as depicted at block 222. In certain embodiments, the injection may continue until the clinician manually terminates the injection or modifies the injection settings. For example, the clinician may consider the indication of extravasation (block 218), including any information pertaining to the injection progress, etc., and allow the injection to continue or modify the injection procedure.

In an embodiment where an extravasation is indicated (block 214) and a manual mode is not selected (block 220), the system 10 may automate control of the injection, in certain embodiments, the system 10 may continue or terminate the injection based on various parameters of the injection. For example, in one embodiment, the system 10 may consider the progress of the current injection before modifying or terminating the injection procedure. As depicted at block 224, after an extravasation has been detected and it has been determined that manual mode is not selected (220), the system 10 may determine the stage of the injection. In one embodiment, the stage of the injection may be determined by the percentage of the medical fluid that has been injected. For example, if 60 milliliters of a 100 milliliter injection has been injected, the system may determine that the injection is at a 60% stage. In other embodiments, the stage may be represented by a range of injected volume, the time of the injection, and the like. Accordingly, the system 10 may determine how far the injection has progressed and the amount of medical fluid and/or time needed to complete the desired injection procedure. As depicted at block 226, the system 10 may then compare the stage of the injection (block 224) to a threshold stage. In one embodiment, the system 10 may include a default value for the threshold stage or a user input value for the threshold stage that is used to determine if the stage of the injection is past the threshold stage. For example, the system 10 may include a default threshold stage value including a percentage, volume, time, or the like. Further, embodiments may include thresholds stage values that are set by the user. For example, a user may enter the threshold stage via the user interface 42, 62.

If the system 10 determines that the injection is past the threshold stage, the system 10 may continue the injection, as depicted at block 228. In one embodiment, continuing the injection may include returning to block 206 of FIG. 4 and continuing to acquire an injection acoustic measurement and proceeding through the steps at blocks 208, 210 and 214 to monitor the injection process. If the system 10 determines that the injection stage is not past the threshold stage (block 226), the system 10 may then proceed to determine if significant extravasation has occurred, as depicted at block 230.

For example, the injection acoustic measurement (block 206) may indicate that only a slight extravasation has occurred and accordingly, the injection may continue, as indicated at block 232. However, if the system 10 determines that a significant extravasation has occurred (e.g., the comparison at block 208 indicates an increased amount of fluid flow in the tissue 44), the system 10 may terminate the injection, as depicted at block 234. In certain embodiments, terminating the injection may include disengaging the drive 38 from the fluid source 36 such that no additional medical fluid is injected into the patient 18. In other embodiments, terminating the injection may include performing a routine to modify the injection protocol such that the rate of injecting the fluid is reduced and or saline may be used to flush the system 10.

Once the system 10 has determined that a significant extravasation has occurred (block 230) and steps have been taken to terminate the injection (block 234), the system 10 may provide an indication of the injection termination, as depicted at block 236. For example, the system 10 may provide a visual or audible signal to the clinician indicating the condition and the action taken in response to the situation.

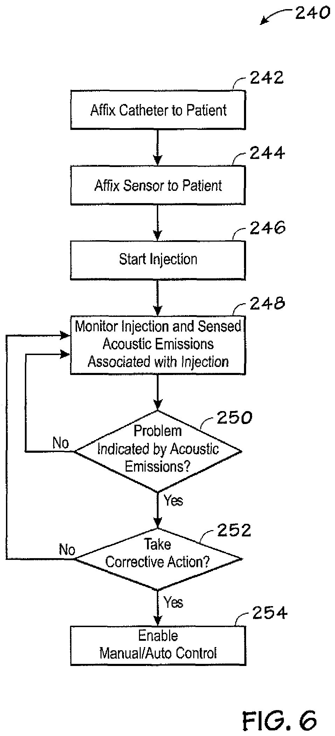

FIG. 6 depicts an exemplary injection procedure 240 that may be performed by a clinician or the like. As depicted at block 242, the clinician may first affix the catheter to the patient. For example, the clinician may insert the needle 28 and/or sheath 26 into the arm and/or vein 32 of the patient 18, as discussed previously. In addition, the clinician may use an adhesive patch 34 to secure the catheter 16 to the patient 18.

Further, the clinician may affix the sensor to the patient, as depicted at block 244. In one embodiment, affixing the sensor 20 to the patient 18 may include strapping the armband 48 to the patient 18 as depicted and discussed previously with regard to FIG. 1. Other embodiments may include affixing the sensor to the patient 18 with the aid of an adhesive or a disposable acoustic matching layer disposed between the sensor and the patient 18, such that the sensor may be reused without the need to be sterilized after each use. As noted previously, the sensor may be positioned at various locations on or near the patient 18. In one embodiment discussed previously with regard to FIG. 2, the steps of affixing the catheter (block 242) and the sensor (block 244) may be accomplished simultaneously. In other embodiments, the steps of affixing the catheter (block 242) and the sensor (block 244) may be accomplished in any order.

After the affixing the catheter and the sensor, the clinician may start the injection, as depicted at block 246. Starting the injection (block 246) may include providing the system 10 with an indication that the patient is ready and, thus, enabling the system 10 to automatically start the injection process. In another embodiment, the clinician may simply press a "start" button or other item on the user interface to enable the drive and initiate the injection.

Once the injection has begun, the clinician may monitor the injection and the sensed acoustic emissions associated with the injection, as indicated at block 248. In one embodiment, monitoring the injection (block 248) may include visually inspecting the injection site 48 and the patient 18 to verify that the catheter 16 remains affixed to the patient 18 and that no visual signs of a complication, such as an extravasation, are present. Further, monitoring the sensed acoustic emissions associated with the injection (block 248) may include the clinician inspecting the user interface for an indication of an extravasation. For example, the clinician may monitor the data processed by the monitor 14 or may monitor visual and audible alerts of the user interface 42, 62.

During the injection process, the clinician may also determine if a problem is indicated by acoustic emissions, as depicted at block 250. In one embodiment, a clinician may evaluate the state of the injection, or may rely on processing to determine that a problem has occurred. If a clinician determines that a problem has not occurred, the clinician may continue to monitor the injection as indicated by the arrow returning to block 248 from block 250.

However, if the clinician determines that a problem exists, the clinician may then make an additional determination as to whether they should take corrective action, as depicted at block 252. For example, the clinician may consider the severity of the problem and/or the stage of the injection process to determine if it is in the best interest of the patient 18 to continue the injection process, or to interrupt the injection process. If a clinician determines that corrective action is not desired, the clinician may continue to monitor the injection as indicated by the arrow returning to block 248 from block 250.

As indicated by block 254, if the clinician determines that corrective action is desired, the clinician may enable manual or automatic control. For example, where the system 10 has detected a problem and alerted the clinician, the clinician may allow the system 10 to continue to take corrective action in an automated procedure, as discussed previously with regard to FIGS, 4 and 5. However, if the problem is not detected by the system 10 or the clinician feels the automated response may not be sufficient, the clinician may take manual control of the system 10. For example, the clinician may manually adjust the parameters of the injection, or may manually terminate the injection. Other embodiments may include the clinician taking any variety of actions to resolve the problem.

## Claims

1. An extravasation detector, comprising:
a plurality of passive acoustic devices (20, 50, 52), wherein said passive acoustic devices are configured to sense acoustic emissions produced by fluid flow during an infusion of a patient with a medical fluid;
wherein the detector is further connected to:
a monitor (14) comprising a memory (60), the memory (60) comprising baseline acoustic emissions representative of acoustic emissions produced by fluid flow within a patient, wherein the monitor (14) is configured to identify an extravasation event using an output from each of said passive acoustic devices (20, 50, 52) and said baseline acoustic emissions from said memory (60).

2. The extravasation detector of claim 1, where said passive acoustic devices (20, 50, 52) each comprise a microphone.

3. The extravasation detector of claim 1 or claim 2, and including means to process the acoustic emissions sensed by said passive acoustic devices (20, 50, 52) to account for background noise.

4. The extravasation detector of any preceding claim, and including means to process the acoustic emissions sensed by said passive acoustic devices (20, 50, 52) to determine the position of an extravasation event.

5. The extravasation detector of any preceding claim, wherein said monitor (14) is configured to receive from said passive acoustic devices (20, 50, 52) representative signals indicative of acoustic emissions sensed by said passive acoustic devices (20, 50, 52).

6. The extravasation detector of claim 5, wherein said monitor (14) is configured to process said signals to determine whether an extravasation event has occurred, is occurring, or may occur.

7. The extravasation detector of any preceding claim, comprising a catheter (16).

8. The extravasation detector of claim 7, wherein the catheter (16) is configured to produce acoustic emissions.

9. The extravasation detector of claim 7 or claim 8, comprising said passive acoustic devices (20, 50, 52) coupled to said catheter (16).

10. The extravasation detector of claim 1, wherein said baseline acoustic emissions comprise a baseline acoustic signature.

11. The extravasation detector of claim 1, wherein said baseline acoustic emissions comprise a plurality of baseline acoustic signatures.

12. The extravasation detector of claim 11, wherein said monitor (14) is configured to compare the sensed acoustic emissions to each of said plurality of baseline acoustic signatures in relation to identifying an extravasation event.

13. The extravasation detector of claim 1, wherein the sensed acoustic emissions and said baseline acoustic emissions are from a common patient.

14. The extravasation detector of claim 13, wherein said baseline acoustic emissions are acquired from a patient proximate and prior to initiating an infusion to this same patient.

15. The extravasation detector of claim 1, wherein said baseline acoustic emissions are based on at least one of normal blood flow, a proper injection, an average over a group of patients, a theoretical model, and a mathematical model.

16. The extravasation detector of claim 1, wherein said baseline acoustic emissions are acquired under known conditions.

17. The extravasation detector of claim 1, wherein said baseline acoustic emissions are representative of an ideal injection process.

18. The extravasation detector of claim 1, wherein said baseline acoustic emissions are representative of an extravasation profile.

19. A medical fluid injector, comprising:
a power injector (12); and
an extravasation detector according to any of claims 1-18.

20. The medical fluid injector of claim 19, wherein the power injector (12) is configured to control the injection of a medical fluid into a patient based on the acoustic emissions from said passive acoustic devices (20, 50, 52).

21. The medical fluid injector of claim 20, wherein the power injector (12) is configured to control the injection of a medical fluid into a patient based on whether an extravasation has occurred, is occurring, or may occur based on the acoustic emissions of said passive acoustic devices (20, 50, 52).

## Patentansprüche

1. Extravasationsdetektor, umfassend:
eine Vielzahl von passiven akustischen Vorrichtungen (20, 50, 52), wobei die passiven akustischen Vorrichtungen dazu ausgelegt sind, akustische Emissionen zu messen, die von einem Fluiddurchfluss in einem Patienten während einer Infusion eines medizinischen Fluids gemessen werden;
wobei der Detektor ferner verbunden ist mit:
einem Monitor (14), der einen Speicher (60) umfasst, wobei der Speicher (60) akustische Baseline-Emissionen umfasst, die für akustische Emissionen repräsentativ sind, die von einem Fluiddurchfluss in einem Patienten erzeugt werden, wobei der Monitor (14) zur Identifizierung eines Extravasationsereignisses unter Verwendung einer Ausgabe von jeder der passiven akustischen Vorrichtungen (20, 50, 52) und den akustischen Baseline-Emissionen aus dem Speicher (60) ausgelegt ist.

2. Extravasationsdetektor nach Anspruch 1, wobei die passiven akustischen Vorrichtungen (20, 50, 52) jeweils ein Mikrofon umfassen.

3. Extravasationsdetektor nach Anspruch 1 oder Anspruch 2, und umfassend Mittel zur Verarbeitung der von den passiven akustischen Vorrichtungen (20, 50, 52) gemessenen akustischen Emissionen zur Berücksichtigung von Hintergrundrauschen.

4. Extravasationsdetektor nach einem der vorhergehenden Ansprüche, und umfassend Mittel zur Verarbeitung der von den passiven akustischen Vorrichtungen (20, 50, 52) gemessenen akustischen Emissionen zur Bestimmung der Position eines Extravasationsereignisses.

5. Extravasationsdetektor nach einem der vorhergehenden Ansprüche, wobei der Monitor (14) dazu ausgelegt ist, von den passiven akustischen Vorrichtungen (20, 50, 52) repräsentative Signale zu empfangen, die auf akustische Emissionen hinweisen, die von den passiven akustischen Vorrichtungen (20, 50 52) gemessen wurden.

6. Extravasationsdetektor nach Anspruch 5, wobei der Monitor (14) zur Verarbeitung der Signale ausgelegt ist, um zu bestimmen, ob ein Extravasationsereignis aufgetreten ist, auftritt oder auftreten kann.

7. Extravasationsdetektor nach einem der vorhergehenden Ansprüche, umfassend einen Katheter (16) .

8. Extravasationsdetektor nach Anspruch 7, wobei der Katheter (16) zur Erzeugung von akustischen Emissionen ausgelegt ist.

9. Extravasationsdetektor nach Anspruch 7 oder Anspruch 8, umfassend die passiven akustischen Vorrichtungen (20, 50, 52), die mit dem Katheter (16) verbunden sind.

10. Extravasationsdetektor nach Anspruch 1, wobei die akustischen Baseline-Emissionen eine akustische Baseline-Signatur umfassen.

11. Extravasationsdetektor nach Anspruch 1, wobei die akustischen Baseline-Emissionen eine Vielzahl von akustischen Baseline-Signaturen umfassen.

12. Extravasationsdetektor nach Anspruch 11, wobei der Monitor (14) dazu ausgelegt ist, die gemessenen akustischen Emissionen mit jeder der Vielzahl von akustischen Baseline-Signaturen in Bezug auf die Identifizierung eines Extravasationsereignisses zu vergleichen.

13. Extravasationsdetektor nach Anspruch 1, wobei die gemessenen akustischen Emissionen und die akustischen Baseline-Emissionen von einem gemeinsamen Patienten stammen.

14. Extravasationsdetektor nach Anspruch 13, wobei die akustischen Baseline-Emissionen von einem Patienten nahe bei und vor der Einleitung einer Infusion an diesem selben Patienten erfasst werden.

15. Extravasationsdetektor nach Anspruch 1, wobei die akustischen Baseline-Emissionen auf mindestens einem, einem normalen Blutfluss, einer richtigen Injektion, einem Durchschnitt über eine Gruppe von Patienten, einem theoretischen Modell und einem mathematischen Modell basieren.

16. Extravasationsdetektor nach Anspruch 1, wobei die akustischen Baseline-Emissionen unter bekannten Bedingungen erfasst werden.

17. Extravasationsdetektor nach Anspruch 1, wobei die akustischen Baseline-Emissionen für ein ideales Injektionsverfahren repräsentativ sind.

18. Extravasationsdetektor nach Anspruch 1, wobei die akustischen Baseline-Emissionen für ein Extravasationsprofil repräsentativ sind.

19. Medizinischer Fluidinjektor, umfassend:
einen Power Injektor (12); und
einen Extravasationsdetektor nach einem der Ansprüche 1-18.

20. Medizinischer Fluidinjektor nach Anspruch 19, wobei der Power Injektor (12) zur Kontrolle der Injektion eines medizinischen Fluids in einen Patienten auf Basis der akustischen Emissionen von den passiven akustischen Vorrichtungen (20, 50, 52) ausgelegt ist.

21. Medizinischer Fluidinjektor nach Anspruch 20, wobei der Power Injektor (12) zur Kontrolle der Injektion eines medizinischen Fluids in eine Patienten auf Basis dessen, ob eine Extravasation aufgetreten ist, auftritt oder auftreten kann, auf Basis der akustischen Emissionen der passiven akustischen Vorrichtungen (20, 50, 52), ausgelegt ist.

## Revendications

1. Détecteur d'extravasation, comprenant :
une pluralité de dispositifs acoustiques passifs (20, 50, 52), lesdits dispositifs acoustiques passifs étant conçus pour détecter des émissions acoustiques produites par un écoulement de fluide au cours d'une perfusion d'un patient avec un fluide médical ;
le détecteur étant en outre connecté à :
un moniteur (14) comprenant une mémoire (60), la mémoire (60) comprenant des émissions acoustiques de référence représentant les émissions acoustiques produites par un écoulement de fluide dans un patient, le moniteur (14) étant conçu pour identifier un événement d'extravasation à l'aide d'une sortie provenant de chacun desdits dispositifs acoustiques passifs (20, 50, 52) et desdites émissions acoustiques de référence provenant de ladite mémoire (60).

2. Détecteur d'extravasation selon la revendication 1, dans lequel lesdits dispositifs acoustiques passifs (20, 50, 52) comprennent chacun un microphone.

3. Détecteur d'extravasation selon la revendication 1 ou la revendication 2, et comprenant des moyens pour traiter les émissions acoustiques détectées par lesdits dispositifs acoustiques passifs (20, 50, 52) pour tenir compte du bruit de fond.

4. Détecteur d'extravasation selon l'une quelconque des revendications précédentes, et comprenant des moyens pour traiter les émissions acoustiques détectées par lesdits dispositifs acoustiques passifs (20, 50, 52) afin de déterminer la position d'un événement d'extravasation.

5. Détecteur d'extravasation selon l'une quelconque des revendications précédentes, dans lequel ledit moniteur (14) est conçu pour recevoir en provenance desdits dispositifs acoustiques passifs (20, 50, 52) des signaux représentatifs indiquant des émissions acoustiques détectées par lesdits dispositifs acoustiques passifs (20, 50, 52).

6. Détecteur d'extravasation selon la revendication 5, dans lequel ledit moniteur (14) est conçu pour traiter lesdits signaux afin de déterminer si un événement d'extravasation s'est produit, est en train de se produire ou risque de se produire.

7. Détecteur d'extravasation selon l'une quelconque des revendications précédentes, comprenant un cathéter (16).

8. Détecteur d'extravasation selon la revendication 7, dans lequel le cathéter (16) est conçu pour produire des émissions acoustiques.

9. Détecteur d'extravasation selon la revendication 7 ou la revendication 8, comprenant lesdits dispositifs acoustiques passifs (20, 50, 52) couplés audit cathéter (16).

10. Détecteur d'extravasation selon la revendication 1, dans lequel lesdites émissions acoustiques de référence comprennent une signature acoustique de référence.

11. Détecteur d'extravasation selon la revendication 1, dans lequel lesdites émissions acoustiques de référence comprennent une pluralité de signatures acoustiques de référence.

12. Détecteur d'extravasation selon la revendication 11, dans lequel ledit moniteur (14) est conçu pour comparer les émissions acoustiques détectées à chaque signature de ladite pluralité de signatures acoustiques de référence en relation avec l'identification d'un événement d'extravasation.

13. Détecteur d'extravasation selon la revendication 1, dans lequel les émissions acoustiques détectées et lesdites émissions acoustiques de référence proviennent d'un patient commun.

14. Détecteur d'extravasation selon la revendication 13, dans lequel lesdites émissions acoustiques de référence sont acquises à partir d'un patient proche et avant de commencer une perfusion de ce même patient.

15. Détecteur d'extravasation selon la revendication 1, dans lequel lesdites émissions acoustiques de référence sont basées sur un écoulement sanguin normal et/ou une injection correcte et/ou une moyenne sur un groupe de patients et/ou un modèle théorique et/ou un modèle mathématique.

16. Détecteur d'extravasation selon la revendication 1, dans lequel lesdites émissions acoustiques de référence sont acquises dans des conditions connues.

17. Détecteur d'extravasation selon la revendication 1, dans lequel lesdites émissions acoustiques de référence sont représentatives d'un processus d'injection idéal.

18. Détecteur d'extravasation selon la revendication 1, dans lequel lesdites émissions acoustiques de référence sont représentatives d'un profil d'extravasation.

19. Injecteur de fluide médical, comprenant :
un injecteur électrique (12) ; et
un détecteur d'extravasation selon l'une quelconque des revendications 1 à 18.

20. Injecteur de fluide médical selon la revendication 19, dans lequel l'injecteur électrique (12) est conçu pour commander l'injection d'un fluide médical dans un patient sur la base des émissions acoustiques provenant desdits dispositifs acoustiques passifs (20, 50, 52).

21. Injecteur de fluide médical selon la revendication 20, dans lequel l'injecteur électrique (12) est conçu pour commander l'injection d'un fluide médical dans un patient selon qu'une extravasation s'est produite, est en train de se produire ou risque de se produire, sur la base des émissions acoustiques provenant desdits dispositifs acoustiques passifs (20, 50, 52).
